# EUROPEAN PATENT APPLICATION

(11) **EP 4 420 519 A1**
(43) Date of publication of application: **28.08.2024**
(21) Application number: 23305223.2
(22) Date of filing: 21.02.2023
(51) Int. Cl.: A01N 63/30, A01P 7/04, C12N 1/14

(54) **METHOD FOR TREATING AND/OR PREVENTING BED BUG INFESTATIONS**

(71) Applicant: Izinovation, 69006 Lyon (FR); INSTITUT NATIONAL DE RECHERCHE POUR L'AGRICULTURE, L'ALIMENTATION ET L'ENVIRONNEMENT, 75007 Paris (FR); Université d'Aix Marseille, 13007 Marseille (FR); Centre National de la Recherche Scientifique, 75016 Paris (FR); Ecole Centrale de Marseille, 13013 Marseille (FR)
(72) Inventor: LEMARIÉ, Emeline, 13640 La Roque d Anthéron (FR); HAON, Mireille, 13011 Marseille (FR); LAFOND, Michael, 83220 Le Pradet (FR); RAMETTI, Lucile, 69960 Corbas (FR); LASSEUR, Romain, 69210 Bully (FR); BERRIN, Jean-Guy, 13360 Roquevaire (FR)
(74) Representative: Germain Maureau

(57) **Abstract**

The present invention relates to a method for treating and/or preventing bed bug infestations which comprises a step of applying at least one entomopathogenic fungus belonging to the species *Metarhizium robertsii* or a composition containing at least one entomopathogenic fungus belonging to the species *Metarhizium robertsii* to a bed bug habitat.

## Description

The present invention relates to a method of treating and/or preventing infestations of bed bugs which involves the use of entomopathogenic fungi or compositions comprising entomopathogenic fungi.

Bed bugs infestation is a common problem in households which is particularly difficult to eradicate. Dwellings (such as homes and hotels) become infested with bed bugs in a variety of ways. Bed bugs and their eggs can be transmitted from other infested dwellings by pets, person's clothing or luggages. Bed bugs often lodge themselves unnoticed in dark crevices, and eggs are nestled in fabric seams. As bed bugs feed on the blood of their host, bed bugs usually remain close to places where potential hosts reside commonly in or near beds or couches of human hosts. Moreover, bed bugs are elusive and usually nocturnal making them hard to spot.

Mechanical approaches to eliminate bed bugs have been explored and include vacuuming up the insects and heat treating or wrapping mattresses.

Moreover, well-known solutions for controlling bed bugs may involve a combination of insecticide and non-insecticide approaches. Nevertheless, bed bugs have become increasingly resistant to insecticides and negative health effects from their use are of concern.

Thus, there is a need to find out new solutions to be used to treat and/or prevent infestations of bed bugs.

The present invention deals with a method, which offers a very efficient solution for treating and/or preventing infestations of bed bugs, in the lodging industry as well as in the residential home, by taking advantage of entomopathogenic fungi which can be horizontally transmitted across bed bugs populations.

In context of the present invention, "horizontal transmission across the bed bugs population" means that the infection by the entomopathogenic fungi will be propagated to not only adult bed bugs but bed bugs of all life stages (i.e., eggs, nymphs, instars and adults) and thus the infestation will be resolved.

In the context of the present invention, the term "treating and/or preventing infestations of bed bugs" refers to preventing infestation, reducing the population of already infested areas, killing or eliminating the bed bugs.

In the context of the present invention, "entomopathogenic fungi" are fungi that are capable of growing, developing, colonizing, destroying, attacking, infecting, killing, disabling, causing disease, and/or causing injury /damage to an insect (i.e., the bed bugs for the present invention), and are thus able to be used in the control insect infestation by adversely affecting the viability or growth of the target insect (i.e., the bed bugs for the present invention).

Therefore, the present invention relates to a method for treating and/or preventing bed bug infestations which comprises a step of applying at least one entomopathogenic fungus belonging to the species *Metarhizium robertsii* or a composition containing at least one entomopathogenic fungus belonging to the species *Metarhizium robertsii* to a bed bug habitat.

In the context of the present invention, a "bed bug habitat" may be a surface of any article. Examples of article are furniture (such as beds, bed box springs, futon box springs, mattresses, chair mattresses, cushions, sofas, futons generally, tables and benches, clothing dressers, lighting fixtures, television), wall material (such as plaster, sheet rock, brick, wood), floor (such as tile, parquet, carpet), drapery windows, temperature regulating devices (such as air-conditioning units, radiators, thermostats, heat pumps, heating units), toilets, sinks, tubs, shower rods, shower basins, doors, any relevant parts of vehicles (such as airplanes, ships) for bed bug habitat. Thus, it means any surface of any article which is a bed bug habitat and where it would be advantageous to apply the above detailed entomopathogenic fungus or the above detailed composition containing said entomopathogenic fungus to treat and/or prevent bed bug infestations.

Preferably, bed bug habitat includes areas where bed bugs are known to congregate (i.e., cracks and crevices in wall material, spaces between floor and wall adjacencies).

The above detailed entomopathogenic fungus or the above detailed composition containing said entomopathogenic fungus may be applied directly to a bed bug habitat or via a bed bug control device. Appropriate bed bug control devices are well-known for a skilled man in the art.

The at least one entomopathogenic fungus may be identical or different strains of *Metarhizium robertsii.*

Preferably, the entomopathogenic fungus is a strain of *Metarhizium robertsii* which comprises both :
- the internal transcribed spacer ("ITS") ribosomal sequence as set forth in SEQ ID NO: 1 and
- the translation elongation factor 1-alpha sequence as set forth in SEQ ID NO: 2.

Preferably, the entomopathogenic fungus is a strain of *Metarhizium robertsii* CIRM-BRFM 2512 (deposited under the Budapest treaty on May 11, 2022 at Collection Nationale de Cultures de Micro-organismes (CNCM) Institut Pasteur, 25 rue du Docteur Roux, Paris, France, as CNCM I-5850).

Preferably, at least one entomopathogenic fungus belonging to the species *Metarhizium robertsii* is in a spore or mycelium form. It means that the method of the present invention is carried out by contacting one or more bed bugs with the spores or mycelium of the at least one entomopathogenic fungus belonging to the species *Metarhizium robertsii.*

The amount of the at least one entomopathogenic fungus belonging to the species *Metarhizium robertsii* may be globally comprised between 10⁶ spores/mL and 10⁸ spores/mL of composition.

Preferably, the entomopathogenic fungus belonging to the species *Metarhizium robertsii* is horizontally transmissible across a population of bed bugs.

The entomopathogenic fungus belonging to the species *Metarhizium robertsii* may treat and/or prevent bed bugs infestations in at least one life stage of bed bugs. More precisely, the entomopathogenic fungus belonging to the species *Metarhizium robertsii* may treat and/or prevent bed bugs at the egg stage, the nymph stage, the instar stage, the adult stage, or any combinations thereof.

In embodiments of the invention, the method for treating and/or preventing bed bug infestations comprises :
- a step of applying the at least one entomopathogenic fungus belonging to the species *Metarhizium robertsii* in combination with at least one another entomopathogenic fungus, or
- a step of applying a composition containing the at least one entomopathogenic fungus belonging to the species *Metarhizium robertsii* in combination with at least one another entomopathogenic fungus,
to the bed bug habitat.

The at least one another entomopathogenic fungus may be identical or different strains of said another entomopathogenic fungus.

The at least one another entomopathogenic fungus may be selected in the group consisting of species of *Aegerita, Ascophaera, Aschersonia, Akanthomyces, Aspergillus, Beauveria, Blastodendrion, Calonectria, Coelomycidium, Coelemomyces, Conidiobolus, Cordyceps, Couchia, Culicinomyces, Engyodontium, Entomophaga, Entomophthora, Erynia, Eryniopsis, Filariomyces, Filobasidiella, Fusarium, Gibellula, Hesperomyces, Hirsutella, Hymenostilbe, Hypocrella, Isaria, Lagenidium, Leptolegnia, Nectria, Neozygites, Nomuraea, Massospora, Meristacrum, Metacordyceps, Metarhizium, Metschnikowia, Mycoderma, Myiophagus, Myriangium, Ophiocordyceps, Paecilomyces, Pandora, Paraisaria, Pleurodesmospora, Pochonia Podonectria, Polycephalomyces, Pseudogibellula, Septobasidium, Sorosporella, Sporodiniella, Si-illbella, Trenomyces, Tetranacrium, Tilachlidium, Tolypocladium, Torrubiella, Uredinella, Verticillium, Zoophthora,*

Preferably, the at least one another entomopathogenic fungus may be selected in the group consisting of *Isaria, Metacordyceps, Fusarium, Cordyceps, Beauveria, Metarhizium, Hirsutella, Aschersonia, Ophiocordyceps, Paecilomyces* and *Pochonia.*

More preferably, the at least one another entomopathogenic fungus is *Isaria farinosa.*

Most preferably, the at least one another entomopathogenic fungus is a strain of *Isariafarinosa* CIRM- BRFM 1607 (deposited under the Budapest treaty on May 11, 2022 at Collection Nationale de Cultures de Micro-organismes (CNCM) Institut Pasteur, 25 rue du Docteur Roux, Paris, France, as CNCM I-5849).

Preferably, the at least one another entomopathogenic fungus is in a spore or mycelium form. It means that the method of the present invention may be carried out by further contacting one or more bed bugs with the spores or mycelium of the at least one another entomopathogenic fungus.

The amount of the at least one another entomopathogenic fungus may be globally comprised between 10⁶ spores/mL and 10⁸ spores/mL of composition.

The above detailed entomopathogenic fungi (i.e., the at least one entomopathogenic fungus belonging to the species *Metarhizium robertsii* and optionally the at least one another entomopathogenic fungus) may be produced in a liquid culture media or a solid culture media fermentation process.

Fermentation processes may be conducted using conventional fermentation processes in laboratory or industrial fermenters. Such fermentation processes are well known in the art.

Methods for producing spores or mycelium commonly use solid media. For example, entomopathogenic fungi are grown on as potato dextrose agar (PDA) for two-three weeks at room temperatures.

The media may have high carbon and nitrogen concentrations to facilitate higher yields. Not-limiting examples of suitable nitrogen sources include hydrolyzed casein, yeast extract, hydrolyzed soy protein, hydrolyzed cottonseed protein, and hydrolyzed corn gluten protein. Not-limiting examples of suitable carbon sources include carbohydrates, including cellulose, chitin, glucose, fructose, sucrose, and/or glycerol and/or plant biomass such as rice, wheat or barley. Following the fermentation process, the spores or mycelium of the entomopathogenic fungi may be recovered using conventional techniques (for example by filtration, centrifugation), and then they may be used directly from the liquid culture media or they may be subject to a step of purification and/or further processing steps (for example a drying process: air-drying, freeze drying, or spray drying to a low moisture level). After these steps, the entomopathogenic fungi are stored at a suitable temperature (for example room temperature).

The production of the above detailed entomopathogenic fungi is well-known for a skilled man in the art.

Preferably, the at least one another entomopathogenic fungus is horizontally transmissible across a population of bed bugs.

The at least one another entomopathogenic fungus may treat and/or prevent bed bugs in at least one life stage of bed bugs. More precisely, the at least one another entomopathogenic fungus may treat and/or prevent the bed bugs infestations at the egg stage, the nymph stage, the instar stage, the adult stage, or any combinations thereof.

The at least one entomopathogenic fungus belonging to the species *Metarhizium robertsii* and the at least one another entomopathogenic fungus may contact the bed bugs sequentially or simultaneously.

In embodiments of the invention, the at least one entomopathogenic fungus belonging to the species *Metarhizium robertsii* and the at least one another entomopathogenic fungus are applied sequentially to the bed bug habitat.

In embodiments of the invention, the at least one entomopathogenic fungus belonging to the species *Metarhizium robertsii* and the at least one another entomopathogenic fungus are included in separate compositions which are applied sequentially to the bed bug habitat.

In another embodiments of the invention, the at least one entomopathogenic fungus belonging to the species *Metarhizium robertsii* and the at least one another entomopathogenic fungus are applied simultaneously to the bed bug habitat. The at least one entomopathogenic fungus belonging to the species *Metarhizium robertsii* and the at least one another entomopathogenic fungus may be included in a single composition which is applied to the bed bug habitat.

The composition may be of any forms. More precisely, the composition may be in the form of a gel, a foam, a solid (for example a powder, granule or particle) or a liquid.

The composition can be of any form so long as the composition is able to support the desired activity of the entomopathogenic fungi, regardless of their form (i.e., vegetative state or dormant state).

In embodiments of the invention, the composition may comprise :
- the at least one entomopathogenic fungus belonging to the species *Metarhizium robertsii;*
- optionally the at least one another entomopathogenic fungus (as described above);
- a carrier;
- optionally at least one additional ingredient.

The composition may comprise, based on the total weight of said composition:
- between 85.00 wt. % and 99.98 wt. %, preferably 85.00 wt. % to 95.00 wt. %, of the carrier;
- between 0.02 wt. % and 15.00 wt. %, preferably between 5.00 wt. % to 15.00 wt. %, of the at least one entomopathogenic fungus belonging to the species *Metarhizium robertsii* and optionally the at least one another entomopathogenic fungus.

The carrier may be a suspension medium capable of supporting the entomopathogenic fungi as described above. Indeed, the entomopathogenic fungi as described above may be transferable from the carrier to the body of the target bed bugs.

The carrier may be used to provide an environment to support the viability of the entomopathogenic fungi as described above, including by providing the proper environmental conditions and protecting the entomopathogenic fungi from harmful environmental conditions (for example excess oxygen, moisture and/or ultraviolet radiation).

The carrier may also be used to maintain the activity of the entomopathogenic fungi after said entomopathogenic fungi have been applied to the bed bug habitat.

The carrier will be configured to allow the entomopathogenic fungi as described above to remain efficient (i.e., able to be transferred to the body of the bed bug with a degree of lethality).

Unless the composition is generated immediately prior to use, the carrier may be used to maintain the activity of the above detailed entomopathogenic fungi during storage (i.e., for example in a container for the entire shelf-life of the formulated product).

In embodiments of the invention, the carrier may be a liquid. More precisely, it may be an aqueous or a non-aqueous liquid.

In preferred embodiments of the invention, the carrier is a non-aqueous liquid carrier as bed bugs are hydrophobic and therefore, have a relatively low critical surface tension. Thus, the low surface tension of the non-aqueous liquid will make it more likely that the composition will adhere to the body of the bed bugs.

Preferably, the non-aqueous liquid is a biodegradable non-aqueous liquid.

The non-aqueous liquids may be selected in the group consisting of silicone oils, mineral oils, hexylene glycol, glycerol, linoleic acid, oleic acid, and any combinations thereof.

In another embodiments of the composition, the carrier may be a gel comprising at least one liquid and at least one gelling agent.

The liquid may be an aqueous or non-aqueous liquid. As explained above, given of the hydrophobicity of the bed bugs, the liquid is preferably a non-aqueous liquid, and more preferably a biodegradable non-aqueous liquid. The non-aqueous liquid may be selected amongst the above detailed non-aqueous liquids.

The gelling agent of the gel may be any agent capable of dissolving in the liquid phase as a colloid mixture to form a weakly cohesive internal structure.

The gelling agent may be selected in the group consisting of polyvinyl acetate, polyvinyl alcohols, polyvinylpyrrolidones, polyacrylates, copolymers of two or more monomers such as acrylic acid, methacrylic acid, maleic acid, fumaric acid, vinylpyrrolidone, ethylenically unsaturated monomers (ethylene, butadiene, isoprene, chloroprene, styrene, divinylbenzene), vinyl halides, vinyl esters, vinyl methyl ketone or esters of acrylic acid or methacrylic acid with monohydric alcohols or polyols (for example methyl acrylate, methyl methacrylate, ethyl acrylate, ethylene methacrylate, lauryl acrylate, lauryl methacrylate, decyl acrylate, N,N-dimethylamino-ethyl methacrylate, 2-hydroxyethyl methacrylate, 2-hydroxypropyl methacrylate or glycidyl methacrylate), diethyl esters or monoesters of unsaturated dicarboxylic acids, (meth)acrylamido-N-methylol methyl ether, amides or nitriles (for example acrylamide, methacrylamide, N-methylol(meth)acrylamide, acrylonitrile, methacrylonitrile), ethers (for example vinyl butyl ether, vinyl isobutyl ether or vinyl phenyl ether) and combinations thereof.

The gelling agent may further include hydrophobically-modified clays, surface modified silicas, fumed silicas, and any combinations thereof.

For these embodiments of the invention, the gel may comprise, based of the total weight of the gel:
- between 80 wt. % and 99.99 wt. % of the liquid;
- between 0.01 wt. % and 20.00 wt. % of the gelling agent.

In embodiments of the invention, the carrier may be an emulsifiable suspension.

The additional ingredient is physically and/or chemically compatible with the composition.

The additional ingredient may be selected in the group consisting of biologically active ingredients, chemical insecticides, insect growth regulators, rheology modifying agents (for example thickeners), preservatives, colorants, opacifiers, fragrances, fillers, pH adjusting agents, stabilizers, antioxidants, oxygen scavenger, wetting agents UV protectants, fillers, nutritive additives, and any combinations thereof.

Such additional ingredients are known to those skilled in the art.

The biologically active ingredients may be selected in the group consisting of enzymes and microorganisms other than the above described entomopathogenic fungi.

Preferably, the composition comprises at least one enzyme.

In preferred embodiments of the invention, the composition comprises at least one cuticle degrading enzyme. Thus, in these embodiments, the biologically active ingredient is a cuticle degrading enzyme.

In the context of the present invention, a "cuticle degrading enzyme" is an enzyme that is able to at least partially degrade a cuticle of a pest (i.e., a bed bug for the present invention), the epicuticle and/or the procuticle. The cuticle degrading enzyme increases the efficacy of the above described entomopathogenic fungi by increasing the ability of the entomopathogenic fungi to colonize and/or or bore through the bed bug's cuticle to reach the bed bug's body cavity.

The cuticle degrading enzyme may be selected in the group consisting of proteases, peptidases, chitinases, chitosanases, cutinases, lipases, esterases, catalases, oxidases, oxygenases, dehydrogenases, and any combinations thereof.

The composition may further comprise at least one microorganism, other than the above described entomopathogenic fungi. The microorganism can have a variety of beneficial properties for the method of the present invention. For example, the microorganism may be used to :
- reduce odors associated with dead or decaying bed bugs,
- produce enzymes to enhance the activity of the above described entomopathogenic fungi,
- produce or express toxins which supplement and/or enhance the activity of the above described entomopathogenic fungi (for example δ-endotoxin, a-exotoxin, β-exotoxin, and any combinations thereof produced by *Bacillus thuringiensis*),
- produce or express CO₂ to attract bed bugs.

Preferably, the microorganism is a bacterium, and more preferably an insecticide bacterium. The insecticide bacterium may be selected in the group consisting of *Enterobacteriaceae, Proteobacteria, Actinobacteria, Firmicutes, Bacteroidetes, Rickettsiae, Mollicutes,* and any combinations thereof.

The microorganism capable of producing CO₂ may be selected in the group consisting of CO₂ producing yeasts, preferably in the group consisting of the yeasts of *Saccharomyces,* and more preferably the yeasts of *Saccharomyces cerevisiae.*

The composition may further comprise at least one rheology modifying agent. Preferably, the rheology modifying agent may comprise a thickener. The thickener may be selected in the group consisting of polyacrylic acids, polyvinylpyrrolidone homo- or copolymers, polyethylene glycols, ethylene oxide/propylene oxide copolymers, polyvinyl alcohols and non-ionically or ionically modified celluloses, thixotropic xanthan-based thickeners, precipitated or pyrogenic silicas, kaolins, bentonites, aluminum/silicon mixed oxides, silicates, and any combinations thereof.

The composition may further comprise at least one preservative. The preservative may be selected in the group consisting of sodium azide, thimerosol, 2-bromo-2-nitro-1,3-propanadiol, 1-(3-chloroallyl)-3,5,7-triaza-1-azoniaadamantane chloride, dibromonitrilopropionamide, 1,2-benzisothiazolin-3-one, 5-chloro-2-methyl-4-isosthiazolin-3-one, 2-methyl-4-isosthiazolin-3-one, diazolidinyl urea, tris(hydroxymethyl)nitromethane, sodium o-phenylphenate, copper arsenates, and any combinations thereof.

The composition may further comprise at least one antioxidant. For example, the antioxidant may be selected in the group consisting of Vitamin E, α-tocopherol, and any combinations thereof.

The composition may further comprise at least one filler. The filler may be selected in the group consisting of calcium carbonate, ground quartz, and any combinations thereof.

In embodiments of the invention, the composition may further comprise at least one chemical insecticide.

For example, the chemical insecticide may be selected in the group consisting of pyrethroids (such as permetherin, resmethrin, phenothrin, deltamethrin, bioallethrin, D-allethrin, esfenvalerate, tetramethrin, cyphenothrin, imiprothrin, alkyl dimethyl benzyl ammonium chloride, beta-cyfluthrin, prallethrin, bifenthrin, lambda-cyhalothrin, zeta-cypermethrin, gamma-cyhalothrin), organophosphates (such as dichlorvos), pyrethrins, neonicotinoids (such as imidacloprid, acetamiprid, dinotefuran), carbamates (such as propoxur), pyroles (for example chlorfenapyr), and any combinations thereof.

In embodiments of the invention, the chemical insecticide is selected so that it will not immediately kill the bed bugs to ensure the at least one entomopathogenic fungus belonging to the species *Metarhizium robertsii* and optionally the at least one another entomopathogenic fungus will be subsequently horizontally transmitted across the bed bug population.

In other embodiments of the invention, the chemical insecticide is selected so that it will immediately kill the bed bugs and the at least one entomopathogenic fungus belonging to the species *Metarhizium robertsii* and optionally the at least one another entomopathogenic fungus will be horizontally transmitted across the bed bug population by the surviving bed bugs.

In embodiments of the invention, the chemical insecticide is applied to the bed bug habitat during a separate treatment of the method for treating and/or preventing bed bugs infestations of the present invention. This separate treatment which involves the use of at least one chemical insecticide may be carried out before or after the method for treating and/or preventing bed bugs infestations of the present invention. For these embodiments of the invention, the chemical insecticide is not included in the above-described composition but included in a separate composition.

Thus, another object of the invention is a method for treating and/or preventing bed bugs infestations which comprises :
- the carrying out of the above described method for treating and/or preventing bed bugs infestations, and
- a separate treatment which involves the use of at least one chemical insecticide to be applied to the bed bug habitat, said separate treatment being carried out before or after said above described method for treating and/or preventing bed bugs infestations.

As explained, the composition of the above-described method for treating and/or preventing bed bugs infestations may comprise or not some chemical insecticides. Thus, in some embodiments of the invention, some chemical insecticides will be used in the composition of the above-described method for treating and/or preventing bed bugs infestations and for the separate treatment too.

To sum up, in the context of the present invention, the at least one chemical insecticide may be applied either simultaneously or sequentially, with the at least one entomopathogenic fungus belonging to the species *Metarhizium robertsii* and optionally the at least one another entomopathogenic fungus as described above.

### DESCRIPTION OF THE SEQUENCES

| Taxonomy | SEQ ID NO | Sequence |
|---|---|---|
| *Metarhizium robertsii* | 1 | GTCGTAACAAGGTCTCCGTTGGTGAACCAGCGGAGGGAT CATTACCGAGTTATCCAACTCCCAACCCCTG TGAATTATACCTTTAATTGTTGCTTCGGCGGGACTTCGCG CCCGCCGGGGACCCAAACCTTCTGAATTTT TTAATAAGTATCTTCTGAGTGGTTAAAAAAATGAATCAAA ACTTTCAACAACGGATCTCTTGGTTCTGGC ATCGATGAAGAACGCAGCGAAATGCGATAAGTAATGTG AATTGCAGAATTCAGTGAATCATCGAATCTTT GAACGCACATTGCGCCCGTCAGTATTCTGGCGGGCATGC CTGTTCGAGCGTCATTACGCCCCTCAAGTCC CCTGTGGACTTGGTGTTGGGGATCGGCGAGGCTGGTTTT CCAGCACAGCCGTCCCTTAAATTAATTGGCG GTCTCGCCGTGGCCCTCCTCTGCGCAGTAGTAAAACACTC GCAACAGGAGCCCGGCGCGGTCCACTGCCG TAAAACCCCCCAACTTTTTATAGTTGACCTCGAATCAGGT AGGACTACCCGCTGAACTTAAGCATATCAA T |
| *Metarhizium robertsii* | 2 | TGGTATCTCCAAGGATGGCCAGACCCGTGAGCACGCTCT CCTCGCCTACACCCTGGGTGTCAAGCAGCTCATTGTCGCC ATCAACAAGATGGACACCACCAAGTGGTCCGAGGCCCGT TACCAGGAAATCATCAAGGAGACTTCCAACTTCATCAAG AAGGTCGGCTACAACCCCAAGACCGTCGCCTTCGTCCCC ATCTCCGGTTTCCACGGTGACAACATGCTTCAGGCCTCCA CCAACTGCCCCTGGTACAAGGGTTGGGAGAAGGAGACC AAGGCTGGCAAGTCCACCGGCAAGACCCTCCTCGAGGCC ATTGACGCCATTGAGCCCCCCAAGCGTCCCACCGACAAG CCCCTCCGTCTTCCCCTCCAGGATGTGTACAAGATTGGCG GTATTGGAACTGTCCCTGTCGGCCGTATCGAGACTGGTG TCCTCAAGCCCGGTATGGTCGTTACCTTCGCTCCCTCCAA CGTCACCACTGAAGTCAAGTCCGTGGAAATGCACCACGA GCAGCTTACCGAGGGTGTCCCCGGTGACAACGTTGGTTT CAACGTGAAGAACGTTTCCGTCAAGGAAATCCGCCGTGG TAACGTTGCTGGTGACTCCAAGAACGACCCCCCCATGGG TGCCGCTTCCTTCGATGCCCAGGTCATCGTTCTCAACCAC CCCGGCCAGGTCGGTGCTGGTTACGCTCCCGTCCTCGATT GCCACACCGCCCACATTGCCTGCAAGTTCTCTGAGATCAA GGAGAAGATTGACCGACGTACCGGTAAGGCTGTTGAGT CTGCCCCCAAGTTCATCAAGTCTGGTGACTCTGCCATCGT CAAGATGGTTCCCTCCAAGCCTATGTGC |

The present invention is illustrated by the following example.

### EXAMPLE

### Mortality of adult bed bugs when exposed to different entomopathogenic fungi:

In this example, the mortality of adult bed bugs when exposed to 13 different strains of entomapathogenic fungi which were :
- sample 1: strain of *Beauveria bassiana* ;
- samples 2 to 4 : 3 differents strains of *Metarhizium robertsii* ;
- sample 5 : strain of *Isaria farinosa*;
- sample 6 : strain of *Metacordyceps chlamydosporia*;
- sample 7: strain of *Fusarium proliferatum*;
- sample 8: strain of *Cordyceps militaris*;
- sample 9 : strain of *Trichothecium roseum*;
- sample 10 : strain of *Gibberella pulicaris*;
- sample 11: strain of *Trichoderma viride*;
- sample 12: strain of *Gibberella pulicaris;*
- sample 13: strain of *Trichothecium* sp.,
has been studied.

The sample 2 of the 1^{st} strain of *Metarhizium robertsii* CIRM-BRFM 2512 was deposited as CNCM I-5850 as mentioned above.

The sample 5 of the strain of *Isariafarinosa* CIRM-BRFM 1607 was deposited as CNCM I-5849 as mentioned above.

The samples 2 to 4 were samples according to the invention and the other samples were comparative samples.

The above detailed strains of entomopathogenic fungi were grown and allowed to sporulate on potato dextrose agar medium plates in closed petri dishes at 25°C for 10 days without controlling the humidity and were stored at 4°C before use.

Inoculum were prepared by recovering the spores from the surface of the mycelium by adding 15 mL of water-Tween 80 and scraping the surface of the mycelium with a rake to the content of 145-mm petri dish cultures of the entomopathogenic fungus (containing fungal biomass [i.e., both mycelia and spores] and potato dextrose agar medium).

300 µL at 10⁷ spores/mL of this homogenate was used to inoculate by spraying the piece of cotton fabric where 10 live adult bed bugs were added (forced contact) for one condition (glass jar with sterile breathable film). Every condition was duplicated and incubated 14 days at room temperature (25°C) with 50-70 % of humidity. Mortality was monitored every day.

The table 1 here below details the average percentage of death of the bed bugs per day for the 13 tested strains of filamentous fungi. The first line of the table 1 represents a negative control, i.e., the result obtained with the above detailed 15 mL mixture of water and Tween 80.

**Table 1: Average percentages of death of the bed bugs per day**

| | **Average % of death/day** | | | | | | | | | | | | |
|---|---|---|---|---|---|---|---|---|---|---|---|---|---|
| **Experiment** | 2 | 3 | 4 | 5 | 6 | 7 | 8 | 9 | 10 | 11 | 12 | 13 | 14 |
| Negative control: H₂O-Tween 80 | 0 | 0 | 0 | 0 | 20 | 30 | 30 | 30 | 30 | 30 | 30 | 30 | 30 |
| Sample 1: *Beauveria bassiana* | 0 | 0 | 0 | 0 | 75 | 80 | 90 | 90 | 90 | **100** | 100 | 100 | 100 |
| Sample 2: 1^{st} strain of *Metarhizium robertsii* CIRM-BRFM 2512 (i.e. deposited as CNCM I-5850) | 0 | 0 | 0 | 63 | **100** | 100 | 100 | 100 | 100 | 100 | 100 | 100 | 100 |
| Sample 3: 2^{nd} strain of Metarhizium robertsii | 0 | 0 | 5 | 75 | **100** | 100 | 100 | 100 | 100 | 100 | 100 | 100 | 100 |
| Sample 4: 3^{rd} strain of *Metarhizium robertsii* | 0 | 0 | 0 | 55 | **100** | 100 | 100 | 100 | 100 | 100 | 100 | 100 | 100 |
| Sample 5: *Isaria farinosa* CIRM-BRFM 1607 (i.e. deposited as CNCM I-5849) | 0 | 0 | 10 | 30 | 90 | 95 | 95 | **100** | 100 | 100 | 100 | 100 | 100 |
| Sample 6: *Metacordyceps chlamydosporia* | 0 | 0 | 0 | 10 | 10 | 15 | 25 | 30 | 55 | 65 | 80 | 85 | **100** |
| Sample 7: *Fusarium proliferatum* | 0 | 0 | 0 | 5 | 15 | 20 | 40 | 45 | 55 | 55 | 60 | 65 | 90 |
| Sample 8: *Cordyceps militaris* | 0 | 0 | 10 | 15 | 15 | 20 | 20 | 25 | 30 | 50 | 70 | 75 | 85 |
| Sample 9: *Trichothecium roseum* | 0 | 0 | 0 | 0 | 15 | 20 | 25 | 30 | 30 | 35 | 40 | 45 | 60 |
| Sample 10: *Gibberella pulicaris* | 0 | 0 | 0 | 0 | 10 | 10 | 15 | 20 | 20 | 20 | 20 | 20 | 25 |
| Sample 11: *Trichoderma viride* | 0 | 0 | 0 | 0 | 0 | 0 | 0 | 0 | 0 | 0 | 10 | 10 | 20 |
| Sample 12: *Gibberella pulicaris* | 0 | 0 | 0 | 0 | 0 | 0 | 0 | 0 | 0 | 0 | 6 | 13 | 13 |
| Sample 13: *Trichothecium sp* | 0 | 0 | 0 | 0 | 5 | 5 | 5 | 5 | 5 | 5 | 5 | 5 | 5 |

In view of the table 1, we note that the exposure of bed bugs to the strains of *Metarhizium robertsii* results in higher mortality rates than the exposure to the strains of the other tested filamentous fungi. Indeed, for the strains of *Metarhizium robertsii,* a mortality of 100% of the bed bugs is reached only after 6 days. In particular, these results obtained with the four different strains of *Metarhizium robertsii* are clearly better than the results obtained with the strain of *Beauveria bassiana,* which is a known entomopathogenic fungus used for eradicating bed bugs (i.e., 6 days versus 11 days, respectively). Moreover, other well-known entomopathogenic fungi (e.g., *Metacordyceps chlamydosporia* and *Cordyceps militaris*) are much less efficient on bedbugs than *Metarhizium robertsii.*

## Claims

1. A method for treating and/or preventing bed bug infestations which comprises a step of applying at least one entomopathogenic fungus belonging to the species *Metarhizium robertsii* or a composition containing at least one entomopathogenic fungus belonging to the species *Metarhizium robertsii* to a bed bug habitat.

2. The method according to claim 1, **characterized in that** the entomopathogenic fungus is a strain of *Metarhizium robertsii* which comprises both :
- the internal transcribed spacer ("ITS") ribosomal sequence as set forth in SEQ ID NO: 1 and
- the translation elongation factor 1-alpha sequence as set forth in SEQ ID NO: 2.

3. The method according to claim 1 or 2, **characterized in that** the entomopathogenic fungus is a strain of *Metarhizium robertsii* CIRM- BRFM 2512 (deposited under the Budapest treaty as CNCM I-5850).

4. The method according to any one of claims 1 to 3, **characterized in that** the at least one entomopathogenic fungus belonging to the species *Metarhizium robertsii* is in a spore or mycelium form.

5. The method according to any one of the preceding claims, **characterized in that** said method comprises :
- a step of applying the at least one entomopathogenic fungus belonging to the species *Metarhizium robertsii* in combination with at least one another entomopathogenic fungus, or
- a step of applying a composition containing the at least one entomopathogenic fungus belonging to the species *Metarhizium robertsii* in combination with at least one another entomopathogenic fungus,
to the bed bug habitat.

6. The method according to the preceding claim, **characterized in that** at least one another entomopathogenic fungus is selected in the group consisting of *Isaria, Metacordyceps, Fusarium, Cordyceps, Beauveria, Metarhizium, Hirsutella, Aschersonia, Ophiocordyceps, Paecilomyces* and *Pochonia.*

7. The method according to any one of the claims 5 to 6, **characterized in that** the composition comprises :
- the at least one entomopathogenic fungus belonging to the species *Metarhizium robertsii;*
- optionally the at least one another entomopathogenic fungus;
- a carrier;
- optionally at least one additional ingredient.

8. The method according to the claim 7, **characterized in that** the composition comprises, based on the total weight of said composition:
- between 85.00 wt. % and 99.98 wt. %, preferably 85.00 wt. % to 95.00 wt. %, of the carrier;
- between 0.02 wt. % and 15.00 wt. %, preferably between 5.00 wt. % to 15.00 wt. %, of the at least one entomopathogenic fungus belonging to the species *Metarhizium robertsii* and optionally the at least one another entomopathogenic fungus.

9. The method according to any one of the claims 7 to 8, **characterized in that** the additional ingredient is selected in the group consisting of biologically active ingredients, chemical insecticides, insect growth regulators, rheology modifying agents, preservatives, colorants, opacifiers, fragrances, fillers, pH adjusting agents, stabilizers, antioxidants, oxygen scavenger, wetting agents UV protectants, fillers, nutritive additives, and any combinations thereof.

10. The method according to the preceding claim, **characterized in that** the biologically active ingredient is a cuticle degrading enzyme.

11. The method according to the preceding claim, **characterized in that** the cuticle degrading enzyme is selected in the group consisting of proteases, peptidases, chitinases, chitosanases, cutinases, lipases, esterases, catalases, oxidases, oxygenases, dehydrogenases, and any combinations thereof.

12. The method according to any one of the claims 9 to 11, **characterized in that** the chemical insecticide is selected in the group consisting of pyrethroids, organophosphates, pyrethrins, neonicotinoids, carbamates, pyroles, and any combinations thereof.

13. A method for treating and/or preventing bed bugs infestations which comprises :
- the carrying out of the method for treating and/or preventing bed bugs infestations according to any one of claims 1 to 12, and
- a separate treatment which involves the use of at least one chemical insecticide to be applied to the bed bug habitat, said separate treatment being carried out before or after said method for treating and/or preventing bed bugs infestations according to any one of claims 1 to 12.
